# FASCICULE DE BREVET EUROPEEN

(11) **EP 2 136 744 B1**
(45) Date de publication et mention de la délivrance du brevet: **05.06.2013**
(21) Numéro de dépôt: 08788158.7
(22) Date de dépôt: 10.04.2008
(51) Int. Cl.: A61F 5/02

(54) **DISPOSITIF DE SOUTIEN LOMBAIRE**
LUMBALE STÜTZVORRICHTUNG
LUMBAR SUPPORT DEVICE

(30) Priorité: 10.04.2007 FR 0702600
(43) Date de publication de la demande: 30.12.2009
(73) Titulaire: THUASNE, 92300 Levallois-Perret (FR)
(72) Inventeur: CONVERT, Reynald, F-42800 SAINT MARTIN LA PLAINE (FR); DE MONCUIT, Henri, F-42000 Saint Etienne (FR); PETIOT, Séverine, F-42230 Roche La Moliere (FR)
(74) Mandataire: Balesta, Pierre
(86) Numéro de dépôt international: PCT/FR2008/050636
(87) Numéro de publication internationale: WO 2008/142331

(56) Documents cités:
- US-A- 2 104 699
- US-A- 3 920 008
- US-A- 4 836 194
- US-B1- 6 766 532

## Description

La présente invention est dans le domaine technique des dispositifs de soutien lombaire.

Habituellement, les dispositifs de soutien lombaire sont formés d'une première ceinture principale disposée directement sur l'usager et venant exercer une pression sur l'abdomen et le dos afin de répartir les tensions et corriger la posture. La première ceinture peut comprendre également un plastron dorsal de renfort améliorant le maintien de la colonne vertébrale. Lorsque l'usager souhaite renforcer la pression exercée par la première ceinture, les dispositifs lombaires comprennent généralement une seconde ceinture venant se fixer sur la première ceinture. La seconde ceinture est soit solidarisée de manière amovible à la première ceinture, soit fixée sur celle-ci au niveau du plastron dorsal. Pour la fixation amovible de la seconde ceinture sur la première ceinture, celle-ci comporte des organes d'attache complémentaires aux organes d'attache de la première ceinture, lesquels sont disposés au niveau de leurs plastrons dorsaux.

Un dispositif de soutien lombaire selon le préamble de la revendication 1 est connu du document US-A-2104699.

Les dispositifs de l'état de la technique ne permettent pas à l'usager de régler facilement le positionnement de la seconde ceinture par rapport à la première ceinture notamment en fonctionnement s'il souhaite améliorer le soutien des zones du dos ou de l'abdomen non soutenues ou insuffisamment soutenues par la première ceinture. De plus, s'il n'a pas besoin d'un soutien complémentaire, la seconde ceinture est gênante et nuit à son confort puisqu'elle est inamovible pour les cas où elle est fixée au plastron dorsal de la première ceinture.

La présente invention a pour but de pallier tout ou partie des problèmes précités. Elle a pour objet un dispositif de soutien lombaire comprenant une ceinture dont les deux extrémités sont pourvues de premiers organes complémentaires d'attache pour sa fermeture et une sangle de rappel dont les deux extrémités sont pourvues de seconds organes d'attaches pour sa fermeture sur elle-même et/ou sur la face extérieure de ladite ceinture. Ce dispositif est caractérisé en ce que la sangle de rappel est solidarisée à la ceinture par des moyens de solidarisation et de blocage aptes à permettre une libre translation verticale de ladite sangle de rappel par rapport à ladite ceinture, moyennant quoi l'usager peut ajuster puis bloquer en translation verticale la position en hauteur de la sangle de rappel selon la hauteur disponible sur la ceinture.

La première ceinture a pour fonction d'exercer une pression suffisante sur le dos et l'abdomen de l'usager afin de limiter les mouvements et transmettre à travers l'abdomen une partie des contraintes mécaniques s'exerçant sur la colonne vertébrale.

On entend par libre translation verticale tout déplacement rectiligne continu ou discontinu dans une direction qui correspond à la position verticale de l'usager, celui-ci étant debout.

La sangle de rappel permet d'augmenter la pression exercée par la ceinture notamment sur des zones de l'abdomen et du dos non soutenues ou insuffisamment soutenues par ladite ceinture. L'usager ajuste le maintien supplémentaire apporté par la sangle de rappel en fonction de son confort et selon sa pathologie, simplement en actionnant les moyens de solidarisation et de blocage en sorte de déplacer en translation verticale la sangle de rappel par rapport à la ceinture puis bloquer la position en hauteur de la sangle de rappel selon la hauteur disponible sur la ceinture de sorte que la sangle de rappel ne puisse plus coulisser verticalement sur la ceinture.

L'action mécanique de la ceinture sur le dos et l'abdomen est fonction de la position de celle-ci le long de la colonne vertébrale et de la pression qu'elle exerce lors de sa mise en tension. Ainsi la sangle de rappel permet de compléter et ajuster la disposition des pressions exercées.

De plus, les seconds organes d'attache de la sangle de rappel étant aménagés à ses extrémités, aucun organe d'attache du type crochets Velcro® n'est disposé sur sa longueur et donc ne risque d'accrocher les vêtements de l'usager lorsque la sangle de rappel déborde au-dessus ou en-dessous de la ceinture.

De préférence, la ceinture et la sangle de rappel sont dans des textiles élastiques facilitant l'évacuation de la chaleur emmagasinée en excès par convection et de la sueur par évaporation.

Dans une variante, les moyens de solidarisation et de blocage sont constitués de deux moyens de coulissement, l'un asservi à la ceinture et l'autre à la sangle de rappel, aptes à permettre un libre coulissement vertical de la sangle de rappel par rapport à ladite ceinture.

Dans une variante, les deux moyens de coulissement coulissent l'un par rapport à l'autre avec un léger frottement, suffisant pour bloquer un premier moyen de coulissement dans une position donnée par rapport au second moyen de coulissement, mais faible de sorte que l'actionnement par l'usager desdits moyens de coulissement ne représente pas un effort important.

Dans une variante, la sangle de rappel est disposée librement dans les moyens de solidarisation et de blocage de sorte qu'elle peut elle-même translater transversalement par rapport auxdits moyens, lors de sa mise en tension en vue de son placement autour de la taille de l'usager.

On entend par translation transversale tout déplacement rectiligne continu ou discontinu dans une direction sensiblement perpendiculaire à la verticale de l'usager, celui-ci étant debout.

Cette disposition donne un degré de liberté supplémentaire à la sangle de rappel. L'usager ajuste facilement la sangle de rappel sur son buste sans modifier la position de la ceinture. La sangle de rappel étant disposée librement dans les moyens de solidarisation et de blocage, il peut éventuellement changer de sangle de rappel selon ses besoins tout en conservant la même ceinture et les mêmes moyens de solidarisation et de blocage.

Dans une variante, les moyens de solidarisation et de blocage comportent des premiers moyens d'attache aptes à coopérer avec des seconds moyens d'attache disposés sur la face extérieure de la ceinture.

L'usager fait ainsi coopérer les premiers moyens d'attache avec les seconds moyens d'attache sur la hauteur disponible sur la ceinture, ce qui permet un premier positionnement de la hauteur de la sangle de rappel sur la hauteur disponible sur la ceinture. Ledit premier positionnement est ensuite ajusté en actionnant les moyens de solidarisation et de blocage en sorte de faire coulisser verticalement et ce de façon continue la sangle de rappel sur la ceinture. Par hauteur disponible sur la ceinture, on comprend la hauteur sur la face extérieure de la ceinture selon laquelle sont disposés les seconds moyens d'attache.

Avantageusement, les moyens de solidarisation et de blocage étant amovibles, la sangle de rappel l'est également. L'usager peut donc utiliser soit la ceinture et la sangle de rappel soit uniquement la ceinture sans être gêné, contrairement aux dispositifs de l'état de l'art antérieur dans lesquels une seconde ceinture est fixée au plastron dorsal de la première ceinture.

Dans une variante, les moyens de coulissement comprennent au moins un jeu de deux passants, à savoir un premier passant vertical de hauteur intérieure H, formé par une partie avant et une partie arrière entre lesquelles passe la sangle de rappel, et un second passant, solidarisé à la ceinture, de hauteur intérieure h inférieure à H, formé par une partie avant et une partie arrière entre lesquelles passe la partie arrière du premier passant de sorte que le premier passant peut coulisser verticalement à l'intérieur du second passant sur une distance D égale à H - h.

Etant donné que le premier passant reçoit la sangle de rappel, la mise en coulissement vertical du premier passant dans le second passant entraîne corrélativement la sangle de rappel dans un mouvement de translation verticale.

Les premier et second passants sont conçus en sorte que des forces de frottement s'opposent au libre coulissement vertical et continu du premier passant dans le second passant. Lesdites forces de frottement sont faibles pour ne pas nécessiter d'efforts importants pour l'usager lors de l'actionnement des moyens de solidarisation et de blocage, mais elles sont suffisantes pour faire office de moyens de blocage bloquant le coulissement vertical du premier passant par rapport au second passant lorsque l'usager n'actionne plus lesdits moyens de solidarisation et de blocage.

De préférence, la sangle de rappel n'est pas fixée mais est disposée librement entre les parties avant et arrière dudit premier passant de sorte qu'elle peut elle-même translater transversalement de façon continue dans ledit premier passant, soit lors de sa mise en tension soit lorsqu'elle est retirée si l'usager n'en a pas besoin ou s'il veut changer de sangle de rappel, tout en conservant le jeu de passants pour sa fixation amovible sur la ceinture.

Le dispositif de soutien lombaire selon la présente invention comprend autant de jeux de deux passants que nécessaire facilitant le coulissement vertical et le blocage de la sangle de rappel sur la hauteur de la ceinture correspondant à la distance D égale à H - h.

Dans une variante, le premier passant comporte à chacune des ses extrémités une languette de préhension dont peut se saisir l'usager pour réaliser le coulissement vertical de la sangle de rappel.

Cette disposition rend plus facile pour l'usager l'ajustement en hauteur de la sangle de rappel par rapport à la ceinture.

Dans une variante, les premiers moyens d'attache sont disposés sur ou forment la face extérieure du second passant.

Dans une variante, des seconds moyens d'attache sont disposés sur tout ou partie de la hauteur de la ceinture, notamment au niveau du plastron dorsal, de sorte que les moyens de solidarisation et de blocage peuvent être fixés de manière amovible à différentes hauteurs sur la ceinture.

Grâce à cette disposition, l'usager dispose d'une plus grande amplitude d'ajustement en hauteur de la sangle de rappel par rapport à la ceinture, combinant d'une part la hauteur autorisée par la libre translation verticale et continue, par exemple dans le cas de l'emploi d'un jeu de passants de l'ordre de D=H-h, et d'autre part la variation possible de positionnement du second passant sur la hauteur du plastron dorsal de la ceinture. Ainsi, il est possible que la sangle de rappel soutienne des zones du corps non soutenues par la ceinture soit en périphérie supérieure soit en périphérie inférieure de ladite ceinture.

Dans une variante, le second passant est formé dans la face extérieure de la ceinture, la partie avant dudit second passant étant délimitée entre deux ouvertures superposées verticalement, espacées d'une hauteur de l'ordre de h et ménagées dans la face extérieure de la ceinture, ouvertures entre lesquelles passe la partie arrière du premier passant de sorte que le premier passant peut coulisser verticalement à travers lesdites deux ouvertures sur une distance D égale à H - h.

La partie arrière du second passant est considérée dans cette disposition comme formée par la face extérieure de la ceinture et ce de part et d'autre des dites ouvertures. En fonctionnement, la hauteur de la sangle de rappel par rapport à la ceinture est ajustée en faisant coulisser verticalement la partie arrière du premier passant entre les parties avant et arrière du second passant. Dans cette disposition, le jeu de passants étant fixé à la ceinture, son positionnement ne peut être ajusté sur la hauteur de celle-ci.

La sangle de rappel étant disposée librement dans les moyens de solidarisation et de blocage, l'usager peut désolidariser la sangle de rappel de la ceinture et ne conserver attachés à lui que la ceinture et tout ou partie des moyens de solidarisation et de blocage.

Dans une variante, lesdits moyens de solidarisation et de blocage comprennent :
a) au moins un passant vertical, de hauteur intérieure P, formé d'une partie avant et d'une partie arrière, entre lesquelles est disposée librement la sangle de rappel,
b) des premiers moyens d'attache, disposés sur la face de ladite partie avant intérieure audit passant,
c) des seconds moyens d'attache, disposés sur la face de ladite partie arrière intérieure audit passant.

De plus, lesdits premiers et seconds moyens d'attache sont aptes à coopérer pour, dans une position inactive, permettre la libre translation verticale et continue de la sangle de rappel sur toute la hauteur intérieure P du passant et, dans une position active, empêcher au moins en partie cette translation verticale tout en autorisant sa translation transversale.

Dans la position active, lesdits premiers et seconds moyens d'attache coopèrent entre eux sur toute la hauteur intérieure P du passant vertical à l'exception de la zone dans laquelle se trouve la sangle de rappel en sorte de bloquer le coulissement vertical de ladite sangle de rappel selon la hauteur disponible P sur la ceinture. La sangle de rappel ne comporte pas sur sa longueur, de moyens susceptibles d'entraver la translation dans le passant. Ainsi, l'usager peut très facilement ajuster la position en hauteur et en largeur de ladite sangle par rapport à la ceinture.

Dans une sous-variante, la partie arrière du passant est formée par la face extérieure de la ceinture. Ainsi, les seconds moyens d'attache sont disposés sur la face extérieure de la ceinture.

Dans une variante, les premiers et seconds moyens d'attache sont des systèmes d'attache du type à crochets et à boucles ou à boutons pressions ou à aimants ou à adhésifs repositionnables.

De préférence les systèmes d'attache à boucles et à crochets sont du type astrakan et crochets.

Le terme astrakan désigne habituellement une surface tissée ayant été brossée pour former des boucles. Ce type de fermeture peut être utilisée des milliers de fois sans perdre ses performances.

Dans une variante, la ceinture comporte des éléments de renforts, tels que des baleines en acier ressort ou en plastique, formant ou comprenant sur leurs faces extérieures les seconds moyens d'attache.

Ces éléments de renfort améliorent le maintien de la colonne vertébrale apporté par la ceinture.

Dans une variante, les premiers et seconds organes d'attache sont du type à crochets et à boucles, notamment du type astrakan et crochets.

La présente invention sera mieux comprise à la lecture de deux exemples de réalisation, cités à titre non limitatif, et illustrés dans les figures ci-après, annexées à la présente, dans lesquelles :
- la figure 1 est une vue de la face extérieure d'un premier exemple de dispositif de soutien lombaire selon la présente invention ;
- la figure 2 est une vue de la face interne du dispositif de soutien lombaire représenté à la figure 1 ;
- la figure 3 est une vue selon le plan de coupe III-III à la figure 1;
- la figure 4 est une vue de dos de l'usager portant le dispositif de soutien lombaire représentés aux figures 1, 2 et 3 ;
- la figure 5 est une vue de la face extérieure d'un second exemple de dispositif de soutien lombaire selon la présente invention ;
- la figure 6a est une vue selon le plan de coupe VI-VI à la figure 5 du dispositif dans une position inactive ;
- la figure 6b est une vue selon le plan de coupe VI-VI à la figure 5 du dispositif dans une position active.

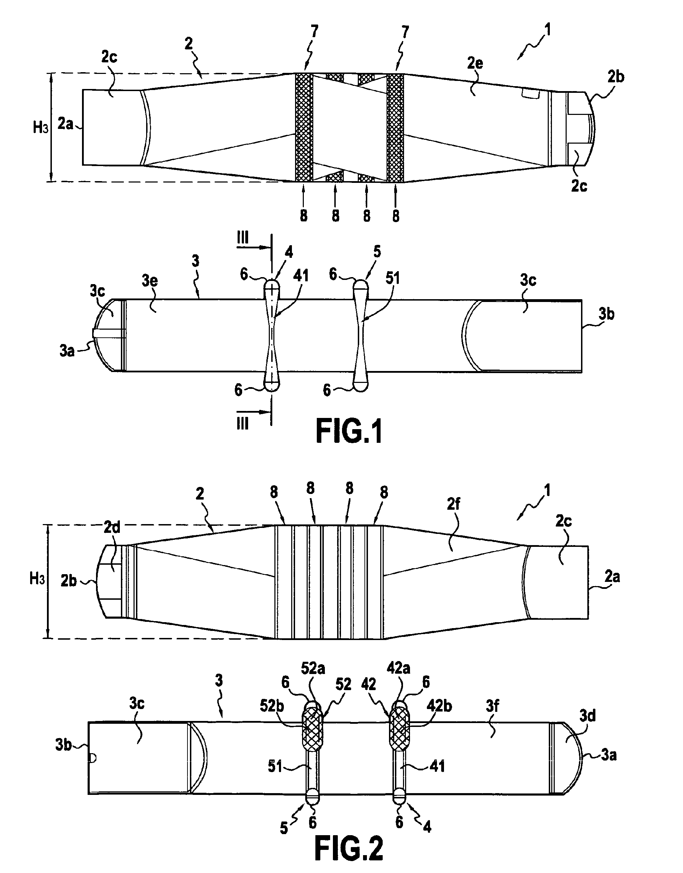

Le premier exemple de dispositif de soutien lombaire 1 est représenté sur sa face extérieure à la figure 1, c'est-à-dire tel qu'il est visible lorsqu'il est porté. Le dispositif 1 comprend une ceinture 2, une sangle de rappel 3 et deux jeux de passants 4 et 5.

La ceinture 2 comprend à ses deux extrémités 2a et 2b des premiers organes d'attache complémentaires 2c et 2d pour sa fermeture sur elle-même autour de la taille de l'usager, de préférence du type à crochets et à boucles, et plus particulièrement à crochets et à astrakan. On désigne par le terme astrakan, toute surface textile, en général un tissu, ayant été brossé pour la formation de boucles aptes à coopérer avec des crochets du type Velcro®.

La sangle de rappel 3 comprend également à ses deux extrémités 3a et 3b des seconds organes d'attache complémentaires 3c et 3d pour sa fermeture sur elle-même autour de la taille de l'usager ou éventuellement sur la face extérieure 2e de la ceinture 2. Les dits seconds organes d'attache sont du type à boucles et à crochets, et de préférence à astrakan et à crochets. Les seconds organes d'attache femelles à boucles 3c sont disposés sur la face extérieure 3e des extrémités 3a et 3b ainsi que sur la face intérieure 3f de l'extrémité 3c. La face intérieure 3f de l'extrémité 3a comporte des seconds organes d'attache mâles 3d à crochets. La sangle de rappel 3 aurait pu comprendre également des seconds organes d'attache par exemple des crochets sur sa face intérieure 3f aptes à coopérer avec des boucles formées sur la face extérieure 2e des extrémités de la ceinture 2. Les premiers organes d'attache 2c de la ceinture 2, sont de préférence des boucles, et sont disposés sur la face extérieure 2e des deux extrémités 2a et 2b et sur la face intérieure 2f de l'extrémité 2c. Les premiers organes d'attache 2d sont de préférence des crochets et sont disposés sur la face interne 2f de l'extrémité 2b.

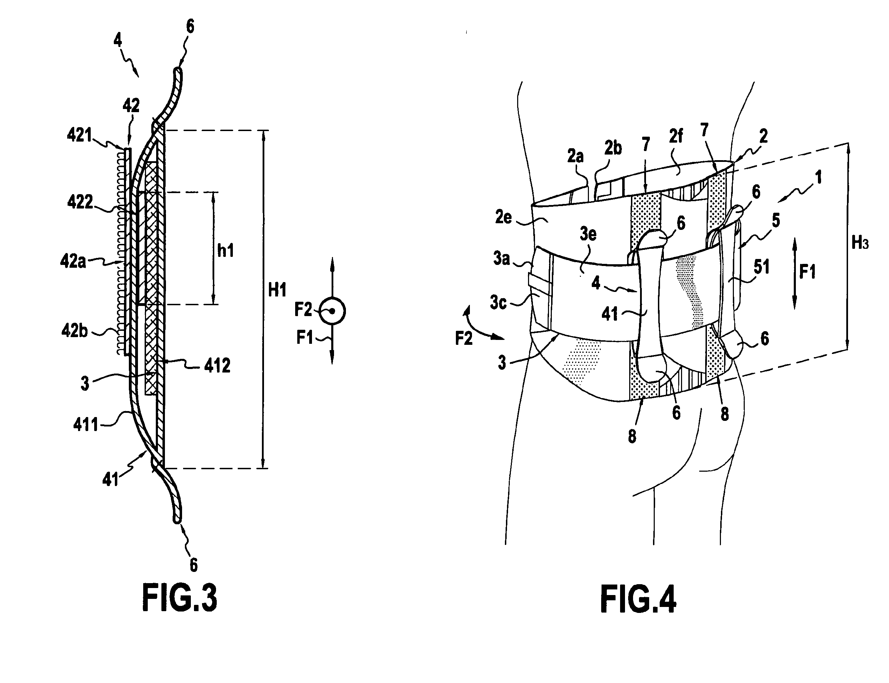

La sangle de rappel 3 est solidarisée et bloquée à la ceinture 2 par deux jeux de deux passants 4 et 5. Chaque jeu de deux passants 4,5 comprend un premier passant vertical 41,51 de hauteur intérieure respectivement H1 et H2, formé par une partie arrière 411,511 et une partie avant 412,512 entre lesquelles passe la sangle de rappel 3, et un second passant 42,52, de hauteur intérieure respectivement h1 et h2 inférieure à H1 et H2, formé par une partie arrière 421,521 et une partie avant 422,522 entre lesquelles passe la partie arrière 411,511 du premier passant 41,51. Dans cet exemple, les dimensions des premiers 41,51 et seconds passants 42,52 étant identiques, mais ceci n'est pas strictement obligatoire, seul le jeu de passant 4 est représenté en coupe à la figure 3. La sangle de rappel 3 est disposée librement dans les premiers passants 41,51. Les premiers passants 41,51 comprennent à chacune de leurs extrémités des languettes de préhension 6 facilitant leur manipulation et leur accès par l'usager lorsque le dispositif est porté.

Les faces extérieures 42a,52a des seconds passants 42,52 comportent des premiers moyens d'attache 42b,52b aptes à coopérer avec des seconds moyens d'attache 7 formée sur la face extérieure 2e de la ceinture 2 , par exemple sur toute la hauteur de caches baleines 8, qui recouvrent des baleines en plastique ou en acier, servant de renfort à la ceinture. Lesdits premiers et seconds moyens d'attache sont de préférence du type à crochets et à boucles, et particulièrement à crochets et à astrakan. De préférence, lesdits seconds moyens d'attache 7 sont des boucles.

Ainsi chaque jeu de passant 4,5 a une fonction de solidarisation et de blocage de la sangle de rappel 3 sur la ceinture 2. La sangle de rappel 3 ne comportant pas de seconds organes d'attache sur sa longueur excepté à ses extrémités 3a et 3b, notamment du type crochets, elle ne risque pas d'accrocher les vêtements de l'usager si elle dépasse de la ceinture 2.

En fonctionnement, l'usager positionne en hauteur la ceinture 2 sur sa colonne vertébrale puis fait coopérer lesdits premiers organes d'attache complémentaires 2c et 2d les uns avec les autres pour sa fermeture. Il positionne ensuite les faces extérieures 42a,52a des seconds passants 42,52 en appliquant les premiers moyens d'attache 42b,52b à une hauteur donnée des caches baleines 8 pourvus des seconds moyens d'attache 7 représentant dans ce cas particulier la hauteur disponible H3 sur la ceinture 2, obtenant ainsi la solidarisation sur la ceinture 2 des deux jeux de passants 4,5 et corrélativement de la sangle de rappel 3. Ce faisant, l'usager obtient un premier réglage en hauteur des jeux de passants 4 et 5 et de la sangle de rappel 3. Il vient ensuite ajuster plus finement la hauteur de la sangle de rappel 3 par rapport à la ceinture 2 en tirant sur les languettes de préhension 6 vers le haut ou le bas pour faire coulisser verticalement, de façon continue, les premiers passants 41,51 dans les seconds passants 42,52 et par là même la sangle de rappel 3 par rapport à la ceinture 2. L'usager dispose pour ce second régalage d'une précision qui est fonction de la différence de hauteur entre la hauteur intérieure H1 et H2 respectivement des premiers passants 41,51 et la hauteur intérieure respectivement h1 et h2 des parties avants 422,522 des seconds passants 42,52.

Les premiers passants 41,51 sont conçus en sorte que des forces de frottement s'opposent au libre coulissement vertical des premiers passants 41,51 dans les seconds passants 42,52. Lesdites forces de frottement sont suffisantes pour faire office de moyens de blocage bloquant le coulissement vertical des premiers passants 41,51 par rapport aux seconds passants 42,52 selon la hauteur disponible égale à la différence de hauteur entre la hauteur intérieure H1,H2 des premiers passants 41,51 et la hauteur intérieure h1,h2 des seconds passants 42,52 lorsque l'usager n'actionne plus les languettes de préhension 6. Les forces de frottement sont cependant faibles pour ne pas nécessiter d'effort important de la part de l'usager lors de l'actionnement des moyens de solidarisation et de blocage 4,5 via les languettes de préhension 6.

Le réglage de la hauteur de la sangle de rappel 3 par rapport à la ceinture 2 peut être réalisé que les extrémités 3a et 3b de ladite sangle 3 soient fermées autour de la taille de l'usager ou non. La sangle de rappel 3 permet d'augmenter la pression exercée par la ceinture 2 lorsqu'elle soutient les mêmes régions de la colonne vertébrale que la ceinture 2 et elle permet de compléter le maintien de la ceinture 2 en exerçant une pression sur des zones non soutenues par celle-ci.

Dans un exemple précis de réalisation donné à titre indicatif : la largeur de la sangle de rappel 3 est de l'ordre de 125 mm, la hauteur intérieure H1, H2 des premiers passants 41,51 est de l'ordre de 135 mm et la hauteur intérieure h1, h2 des parties avants 422,522 des seconds passants 42,52 est de l'ordre de 50 mm. L'usager bénéficie donc d'une amplitude de l'ordre de 75 mm pour faire coulisser verticalement la sangle de rappel 3 par rapport à la ceinture 2.

Par ailleurs la sangle de rappel 3 étant disposée librement dans les premiers passants 41 et 51, l'usager peut facilement la faire translater transversalement de façon continue, ce qui lui procure un degré de liberté supplémentaire dans l'ajustement de la pression qu'il souhaite qu'elle exerce.

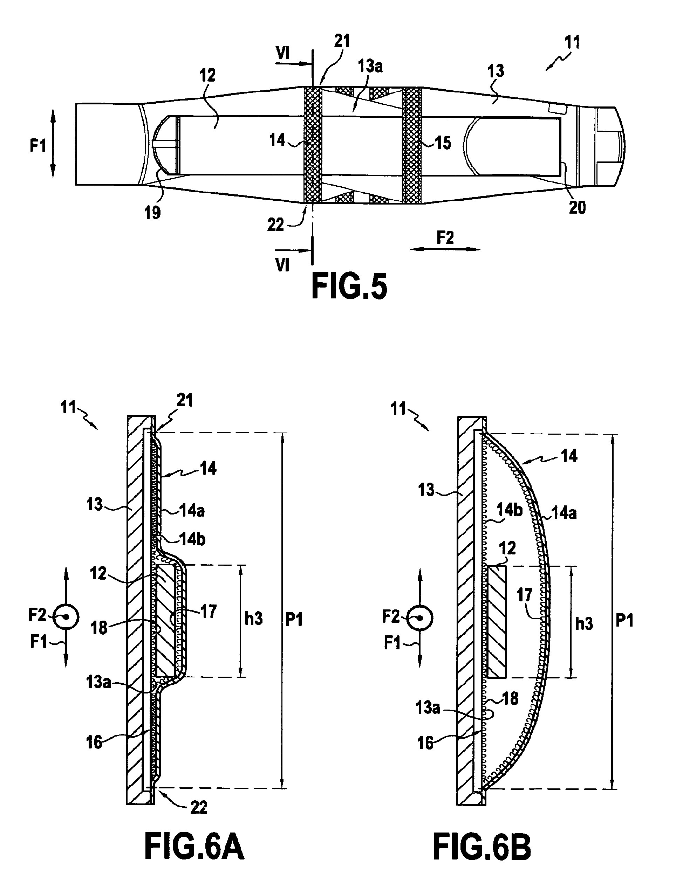

Le second exemple de dispositif de soutien lombaire 11 représenté à la figure 5, diffère du premier exemple en ce que les moyens de solidarisation et de blocage de la sangle de rappel 12 à la ceinture 13 comprennent deux passants verticaux 14 et 15 de hauteur intérieure respectivement P1 et P2. Dans cet exemple, les hauteurs P1 et P2 sont sensiblement égales, mais ceci n'est pas strictement obligatoire. Seul le passant 14 est représenté en coupe aux figures 6a et 6b. Le passant 15 a une structure identique à celle du passant 14. Aux figures 6a et 6b, le passant 14 est formé d'une partie avant 14a et d'une partie arrière 14b formée par la face extérieure 13a de la ceinture 13, laquelle face extérieure 13a recouvre un élément de renfort 16, notamment une baleine en acier ressort. Des premiers moyens d'attache 17 sont disposés sur la face de la partie avant 14a intérieure du passant 14 et des seconds moyens d'attaches 18 sont disposés sur la face extérieure 13a de la ceinture 13. Les premiers 17 et seconds 18 moyens d'attache représentés aux figures 5, 6a et 6b sont du type à boucles et à crochets ; les boucles notamment du type astrakan sont disposées sur la face extérieure 13a de la ceinture 13 et les crochets sont disposés sur la face de la partie avant intérieure 14a du passant 14. En fonctionnement lorsque les moyens de solidarisation et de blocage sont dans une position inactive (figure 6b) la partie avant 14 du passant 14 est écartée de la face extérieure 13a de la ceinture, la sangle de rappel 12 étant disposée librement dans l'espace ainsi délimité. L'usager peut ajuster la position en hauteur de la sangle de rappel 12 par rapport à la ceinture 13, plus particulièrement selon les hauteurs disponibles P1 et P2, par translation verticale continue selon la direction F1 et/ou translation transversale continue selon la direction F2. Une fois la sangle de rappel 12 correctement ajustée à sa taille, l'usager actionne les seconds organes d'attache 19,20 pour sa fermeture sur elle-même ou sur la ceinture 13 puis engage les premiers moyens 17 d'attache à coopérer avec les seconds 18 moyens d'attache sur toute la hauteur intérieure P1 ou P2 excepté sur la hauteur h3 de la sangle de rappel 12 pour empêcher, bloquer la translation verticale de la sangle de rappel 12 tout en autorisant son éventuelle translation transversale et ce de façon continue. La sangle de rappel ne comporte pas de préférence sur sa longueur d'organes d'attache, lesquels empêcheraient une translation transversale dans les passants 14 et 15.

La partie avant 14a du passant 14 est cousue à ses deux extrémités aux bords longitudinaux opposés de la ceinture 13 selon les lignes de couture 21 et 22.

Dans un exemple précis de réalisation donné à titre indicatif : la largeur de la sangle de rappel 12 est de l'ordre de 125 mm, la hauteur intérieure P1 et P2 des passants 14 et 15 est de l'ordre de 235 mm. L'usager bénéficie donc d'une amplitude de l'ordre de 125 mm pour faire translater verticalement la sangle de rappel 12 par rapport à la ceinture 13.

Dans une variante de réalisation, non représentée, du premier exemple de dispositif de soutien lombaire 1 représenté aux figures 1 à 4, le premier jeu de passant 4 est modifié en ce que la partie avant 422 du second passant vertical 42 est formée dans la face extérieure 2e de la ceinture 2, et de préférence dans la face extérieure 2e formant un des cache-baleines 8. La partie avant 422 du second passant 42 est délimité par deux ouvertures horizontales réalisées dans la face extérieure 2e, les dites ouvertures étant superposées verticalement et espacées selon une hauteur de l'ordre de h1. La partie arrière 421 du second passant est ici formée par la face extérieure de la ceinture de part et d'autre desdites deux ouvertures. En fonctionnement la partie arrière 411 du premier passant 41 coulisse verticalement selon la direction F1 entre les dites ouvertures de préférence au regard d'un élément de renfort tel qu'une baleine. La sangle de rappel 3 translate transversalement entre la partie avant 412 du premier passant 41 et la partie avant 422 du second passant 42. Dans cette dernière variante, les moyens de coulissement formés du jeu de passant 4 modifié ne peuvent pas être rapportés amoviblement sur la face extérieure 2e de la ceinture 2. Le dispositif peut comprendre autant de jeu de passants que nécessaires.

## Revendications

1. Dispositif de soutien lombaire (1,11) comprenant une ceinture (2,13) dont les deux extrémités (2a,2b) sont pourvues de premiers organes complémentaires d'attache (2c,2d) pour sa fermeture et une sangle de rappel (3,12) dont les deux extrémités (3a,3b) sont pourvues de seconds organes d'attaches (3c,3d) pour sa fermeture sur elle-même et/ou sur la face extérieure (2e,13a) de ladite ceinture (2,13), **caractérisé en ce que** la sangle de rappel (3,12) est solidarisée à la ceinture (2,13) par des moyens de solidarisation et de blocage (4,5,14,15) en translation verticale de ladite sangle de rappel selon une hauteur disponible sur la ceinture aptes à permettre une libre translation verticale de ladite sangle de rappel (3,12) par rapport à ladite ceinture (2,13), moyennant quoi l'usager peut ajuster par translation verticale de la sangle de rappel puis bloquer la position en hauteur de la sangle de rappel (3,12) selon la hauteur disponible (H3,P1,P2, H1-h1,H2-h2) sur la ceinture (2,13).

2. Dispositif de soutien lombaire (1) selon la revendication 1, **caractérisé en ce que** les moyens de solidarisation et de blocage (4,5) sont constitués de deux moyens de coulissement (41,51,42,52), l'un asservi à la ceinture (2) et l'autre à la sangle de rappel (3), aptes à permettre un libre coulissement vertical de la sangle de rappel (3) par rapport à ladite ceinture (2).

3. Dispositif de soutien lombaire (1) selon la revendication 2, **caractérisé en ce que** les deux moyens de coulissement (41,51,42,52) coulissent l'un par rapport à l'autre avec un léger frottement, suffisant pour bloquer un premier moyen de coulissement dans une position donnée par rapport au second moyen de coulissement, mais faible de sorte que l'actionnement par l'usager desdits moyens de coulissement (41,51,42,52) ne représente pas un effort important.

4. Dispositif de soutien lombaire (1,11) selon l'une des revendications 1 à 3, **caractérisé en ce que** la sangle de rappel (3,12) est disposée librement dans les moyens de solidarisation et de blocage (4,5,14,15) de sorte qu'elle peut elle-même translater transversalement par rapport auxdits moyens (4,5,14,15), notamment lors de sa mise en tension.

5. Dispositif de soutien lombaire (1) selon l'une quelconque des revendications 1 à 4, **caractérisé en ce que** les moyens de solidarisation et de blocage comportent des premiers moyens d'attache (42b,52b) aptes à coopérer avec des seconds moyens d'attache (7) disposés sur la face extérieure (2e) de la ceinture (2).

6. Dispositif de soutien lombaire (1) selon l'une quelconque des revendications 2 à 5, **caractérisé en ce que** les moyens de coulissement (41,51,42,52) comprennent au moins un jeu de deux passants (4,5), à savoir un premier passant vertical (41,51) de hauteur intérieure H (H1,H2), formé par une partie arrière (411,511) et une partie avant (412,512) entre lesquelles passe la sangle de rappel (3), et un second passant (42,52), solidarisé à la ceinture (2), de hauteur intérieure h (h1,h2) inférieure à H (H1,H2), formé par une partie avant (422,522) et une partie arrière (421,521) entre lesquelles passe la partie arrière (411,511) du premier passant (41,51) de sorte que le premier passant (41,51) peut coulisser verticalement à l'intérieur du second passant (42,52) sur une distance D égale à H (H1,H2) - h (h1,h2).

7. Dispositif de soutien lombaire (1) selon la revendication 6, **caractérisé en ce que** le premier passant (41,51) comporte à chacune des ses extrémités une languette de préhension (6) dont peut se saisir l'usager pour réaliser la translation verticale de la sangle de rappel (3).

8. Dispositif de soutien lombaire (1) selon l'une ou l'autre des revendications 5 et 6, **caractérisé en ce que** les premiers moyens d'attache (42b,52b) sont disposés sur ou forment la face extérieure (42a,52a) du second passant (42,52).

9. Dispositif de soutien lombaire (1) selon l'une quelconque des revendications 5 à 8, **caractérisé en ce que** les seconds moyens d'attache (7) sont disposés sur tout ou partie de la hauteur (H3) de la ceinture (2), notamment au niveau du plastron dorsal, de sorte que les moyens de solidarisation (4,5) peuvent être fixés de manière amovible à différentes hauteurs sur la ceinture (2).

10. Dispositif de soutien lombaire (1) selon l'une ou l'autre des revendications 6 et 7, **caractérisé en ce que** le second passant (42,52) est formé dans la face extérieure (2e) de la ceinture (2), la partie avant dudit second passant étant délimitée entre deux ouvertures superposées verticalement et ménagées dans la face extérieure de la ceinture, entre lesquelles passe la partie arrière du premier passant de sorte que le premier passant peut coulisser verticalement à travers lesdites deux ouvertures sur une distance D égale à H - h.

11. Dispositif de soutien lombaire (11) selon l'une ou l'autre des revendications 1 et 4, caractérisé en ce lesdits moyens de solidarisation et de blocage comprennent :
a) au moins un passant vertical (14,15), de hauteur intérieure P (P1,P2), formé d'une partie avant (14a) et d'une partie arrière (14b), entre lesquelles est disposée librement la sangle de rappel (13),
b) des premiers moyens d'attache (17), disposés sur la face de ladite partie (14a) avant intérieure audit passant (14,15),
c) des seconds moyens d'attache (18), disposés sur la face de ladite partie arrière intérieure audit passant (14,15),
et en ce que lesdits premiers (17) et seconds (18) moyens d'attache sont aptes à coopérer pour, dans une position inactive, permettre la libre translation verticale (F1) de la sangle de rappel (12) sur toute la hauteur intérieure P (P1,P2) du passant (14,15) et, dans une position active, empêcher au moins en partie cette translation verticale (F1) tout en autorisant sa translation transversale (F2).

12. Dispositif de soutien lombaire (11) selon la revendication 11, **caractérisé en ce que** la partie arrière du passant (14,15) est formée par la face extérieure (13a) de la ceinture (12).

13. Dispositif de soutien lombaire (1,11) selon l'une quelconque des revendications 5 à 12, **caractérisé en ce que** les premiers (42b,52b,17) et seconds (7,18) moyens d'attache sont des systèmes d'attache du type à crochets et à boucles ou à boutons pressions ou à aimants ou à adhésifs repositionnables.

14. Dispositif de soutien lombaire (1,11) selon l'une quelconque des revendications 5 à 13, **caractérisé en ce que** la ceinture (2,13) comporte des éléments de renforts (8,16), tels que des baleines en acier ressort ou en plastique, formant ou comprenant sur leurs faces extérieures les seconds moyens d'attache (7,18).

15. Dispositif de soutien lombaire (1,11) selon l'une quelconque des revendications 1 à 14, **caractérisé en ce que** les premiers (2c,2d) et seconds (3c,3d) organes d'attache sont du type à crochets et à boucles, notamment du type astrakan et crochets.

## Patentansprüche

1. Lumbale Stützvorrichtung (1, 11), umfassend einen Gurt (2, 13), dessen beiden Enden (2a, 2b) mit ersten komplementären Befestigungselementen (2c, 2d) für seinen Verschluß versehen sind, und einen Rückstellriemen (3, 12), dessen beiden Enden (3a, 3b) mit zweiten Befestigungselementen (3c, 3d) für seinen Verschluß auf sich selbst und/oder auf der Außenseite (2e, 13a) des Gurts (2, 13) versehen sind, **dadurch gekennzeichnet, daß** der Rückstellriemen (3, 12) mit dem Gurt (2, 13) durch Verbindungs- und Feststellmittel (4, 5, 14, 15) des Rückstellriemens in vertikaler Translation entlang einer verfügbaren Höhe auf dem Gurt, die geeignet sind, eine freie vertikale Translation des Rückstellriemens (3, 12) in bezug zum Gurt (2, 13) zu ermöglichen, wodurch der Benutzer durch vertikale Translation des Rückstellriemens die Position des Rückstellriemens (3, 12) in der Höhe entlang der verfügbaren Höhe (H3, P1, P3, H1-h1, H2-h2) auf dem Gurt (2, 13) anpassen und dann feststellen kann.

2. Lumbale Stützvorrichtung (1) nach Anspruch 1, **dadurch gekennzeichnet, daß** die Verbindungs- und Feststellmittel (4, 5) von zwei Gleitmitteln (41, 51, 42, 52) gebildet sind, einem, das dem Gurt (2) zugeordnet ist, und einem anderen, da dem Rückstellriemen (3) zugeordnet ist, die geeignet sind, ein freies vertikales Gleiten des Rückstellriemens (3) in bezug zum Gurt (2) zu ermöglichen.

3. Lumbale Stützvorrichtung (1) nach Anspruch 2, **dadurch gekennzeichnet, daß** die beiden Gleitmittel (41, 51, 42, 52) zueinander mit einer leichten Reibung gleiten, die ausreicht, um ein erstes Gleitmittel in einer gegebenen Position in bezug zum zweiten Gleitmittel festzustellen, aber gering ist, so daß die Betätigung der Gleitmittel (41, 51, 42, 52) durch den Benutzer keine große Kraftanstrengung darstellt.

4. Lumbale Stützvorrichtung (1, 11) nach einem der Ansprüche 1 bis 3, **dadurch gekennzeichnet, daß** der Rückstellriemen (3, 12) frei in den Verbindungs- und Feststellmitteln (4, 5, 14, 15) angeordnet ist, so daß er sich selbst quer zu den Mitteln (4, 5, 14, 15) insbesondere bei seinem Spannen in Translation bewegen kann.

5. Lumbale Stützvorrichtung (1) nach einem der Ansprüche 1 bis 4, **dadurch gekennzeichnet, daß** die Verbindungs- und Feststellmittel erste Befestigungsmittel (42b, 52b) umfassen, die geeignet sind, mit zweiten Befestigungsmitteln (7) zusammenzuwirken, die auf der Außenseite (2e) des Gurts (2) angeordnet sind.

6. Lumbale Stützvorrichtung (1) nach einem der Ansprüche 2 bis 5, **dadurch gekennzeichnet, daß** die Gleitmittel (41, 51, 42, 52) mindestens einen Satz von zwei Durchführungen (4, 5) umfassen, nämlich eine erste vertikale Durchführung (41, 51) mit der inneren Höhe H (H1, H2), die von einem hinteren Teil (411, 511) und einem vorderen Teil (412, 512) gebildet, zwischen denen der Rückstellriemen (3) durgeführt wird, und eine zweite Durchführung (42, 52), die mit dem Gurt (2) verbunden ist, mit der inneren Höhe h (h1, h2), die kleiner als H (H1, H2) ist, die von einem vorderen Teil (422, 522) und einem hinteren Teil (421, 521) gebildet ist, zwischen denen der hintere Teil (411, 511) der ersten Durchführung (41, 51) verläuft, so daß die erste Durchführung (41, 51) vertikal im Inneren der zweiten Durchführung (42, 52) über eine Distanz D gleich H (H1, H2) - h (h1, h2) gleiten kann.

7. Lumbale Stützvorrichtung (1) nach Anspruch 6, **dadurch gekennzeichnet, daß** die erste Durchführung (41, 51) an jedem ihrer Enden eine Greiflasche (6) umfaßt, die der Benutzer ergreifen kann, um die vertikale Translation des Rückstellriemens (3) durchzuführen.

8. Lumbale Stützvorrichtung (1) nach dem einen oder dem anderen der Ansprüche 5 und 6, **dadurch gekennzeichnet, daß** die ersten Befestigungsmittel (42b, 52b) auf der Außenseite (42a, 52a) der zweiten Durchführung (42, 52) angeordnet sind oder diese bilden.

9. Lumbale Stützvorrichtung (1) nach einem der Ansprüche 5 bis 8, **dadurch gekennzeichnet, daß** die zweiten Befestigungsmittel (7) auf der gesamten oder einem Teil der Höhe (H3) des Gurts (2), insbesondere im Bereich des Dorsalplastrons, angeordnet sind, so daß die Verbindungsmittel (4, 5) abnehmbar in verschiedenen Höhen auf dem Gurt (2) befestigt werden können.

10. Lumbale Stützvorrichtung (1) nach dem einen oder dem anderen der Ansprüche 6 und 7, **dadurch gekennzeichnet, daß** die zweite Durchführung (42, 52) in der Außenseite (2e) des Gurts (2) ausgebildet ist, wobei der vordere Teil der zweiten Durchführung zwischen zwei vertikal übereinander angeordneten und in der Außenseite des Gurts vorgesehenen Öffnungen begrenzt ist, zwischen denen der hintere Teil der ersten Durchführung verläuft, so daß die erste Durchführung vertikal durch die beiden Öffnungen über eine Distanz D gleich H - h gleiten kann.

11. Lumbale Stützvorrichtung (11) nach dem einen oder dem anderen der Ansprüche 1 und 4, **dadurch gekennzeichnet, daß** die Verbindungs- und Feststellmittel folgendes umfassen:
a) mindestens eine vertikale Durchführung (14, 15) mit der inneren Höhe P (P1, P2), die von einem vorderen Teil (14a) und einem hinteren Teil (14b) gebildet ist, zwischen denen der Rückstellriemen (13) frei angeordnet ist,
b) erste Befestigungsmittel (17), die auf der Fläche des vorderen Teils (14a) innen in der Durchführung (14, 15) angeordnet sind,
c) zweite Befestigungsmittel (18), die auf der Seite des hinteren Teils innen in der Durchführung (14, 15) angeordnet sind,
und daß die ersten (17) und zweiten (18) Befestigungsmittel geeignet sind zusammenzuwirken, um in einer inaktiven Position die freie vertikale Translation (F1) des Rückstellriemens (12) auf der gesamten inneren Höhe P (P1, P2) der Durchführung (14, 15) zu ermöglichen, und in einer aktiven Position zumindest teilweise diese vertikale Translation (F1) zu verhindern, wobei die transversale Translation (F2) gestattet ist.

12. Lumbale Stützvorrichtung (11) nach Anspruch 11, **dadurch gekennzeichnet, daß** der hintere Teil der Durchführung (14, 15) von der Außenseite (13a) des Gurts (12) gebildet ist.

13. Lumbale Stützvorrichtung (1, 11) nach einem der Ansprüche 5 bis 12, **dadurch gekennzeichnet, daß** die ersten (42b, 52b, 17) und die zweiten (7, 18) Befestigungsmittel Befestigungssysteme vom Typ mit Haken und Schlaufen oder mit Druckknöpfen oder mit Magneten oder mit wieder anbringbaren Haftbändern sind.

14. Lumbale Stützvorrichtung (1, 11) nach einem der Ansprüche 5 bis 13, **dadurch gekennzeichnet, daß** der Gurt (2, 13) Verstärkungselemente (8, 16), wie Federstahl- oder Kunststoffstäbe, umfaßt, die auf ihren Außenseiten die zweiten Befestigungsmittel (7, 18) bilden oder umfassen.

15. Lumbale Stützvorrichtung (1, 11) nach einem der Ansprüche 1 bis 14, **dadurch gekennzeichnet, daß** die ersten (2c, 2d) und die zweiten (3c, 3d) Befestigungselemente vom Typ mit Haken und Schlaufen, insbesondere vom Typ Astrakan und Haken, sind.

## Claims

1. A lumbar support device (1, 11) comprising a belt (2, 13) having its two ends (2a, 2b) provided with first complementary fastener members (2c, 2d) for closing the belt, and a return strap (3, 12) having its two ends (3a, 3b) provided with second fastener members (3c, 3d) for closing in a loop and/or onto the outside face (2e, 13a) of said belt (2, 13), the device being **characterized in that** the return strap (3, 12) is secured to the belt (2, 13) by securing and blocking means (4, 5, 14, 15) for securing said return strap and blocking it against movement in vertical translation over an available height on the belt, said means being suitable for allowing said return strap (3, 12) to move freely in vertical translation relative to said belt (2, 13), thereby enabling the user to adjust the return strap in vertical translation and then to block the height position of the return strap (3, 12) over the available height (H3, P1, P2, H1-h1, H2-h2) on the belt (2, 13).

2. A lumbar support device (1) according to claim 1, **characterized in that** the securing and blocking means (4, 5) are constituted by two slide means (41, 51, 42, 52), one dependent on the belt (2) and the other on the return strap (3) and suitable for enabling the return strap (3) to slide freely vertically relative to said belt (2).

3. A lumbar support device (1) according to claim 2, **characterized in that** the two slide means (41, 51, 42, 52) slide relative to each other with a small amount of friction, sufficient to block a first slide means in a given position relative to the second slide means, but small enough to ensure that user activation of said slide means (41, 51, 42, 52) does not require a large effort.

4. A lumbar support device (1, 11) according to any one of claims 1 to 3, **characterized in that** the return strap (3, 12) is arranged freely in the securing and blocking means (4, 5, 14, 15) so that it can itself move in translation transversely relative to said means (4, 5, 14, 15), in particular while it is being tensioned.

5. A lumbar support device (1) according to any one of claims 1 to 4, **characterized in that** the securing and blocking means include first fastener means (42b, 52b) suitable for co-operating with second fastener means (7) arranged on the outside face (2e) of the belt (2).

6. A lumbar support device (1) according to any one of claims 2 to 5, **characterized in that** the slide means (41, 51, 42, 52) comprise at least one set of two loops (4, 5), namely a vertical first loop (41, 51) of inside height H (H1, H2) formed by a rear portion (411, 511) and a front portion (412, 512) between which the return strap (3) passes, and a second loop (42, 52) secured to the belt (2) of inside height h (h1, h2) less than H (H1, H2), formed by a front portion (422, 522) and a rear portion (421, 521) between which the rear portion (411, 511) of the first loop (41, 51) passes so that the first loop (41, 51) can slide vertically inside the second loop (42, 52) over a distance D equal to H (H1, H2) - h (h1, h2).

7. A lumbar support device (1) according to claim 6, **characterized in that** the first loop (41, 51) includes a grip tongue (6) at each of its ends that the user can grasp in order to move the return strap (3) in vertical translation.

8. A lumbar support device (1) according to claim 5 or claim 6, **characterized in that** the first fastener means (42b, 52b) are arranged on or form the outside face (42a, 52a) of the second loop (42, 52).

9. A lumbar support device (1) according to any one of claims 5 to 8, **characterized in that** the second fastener means (7) are arranged over all or part of the height (H3) of the belt (2), in particular over its back plate, so that the securing means (4, 5) can be releasably fastened at different heights on the belt (2).

10. A lumbar support device (1) according to claim 6 or claim 7, **characterized in that** the second loop (42, 52) is formed in the outside face (2e) of the belt (2), the front portion of said second loop being defined between two vertically superposed openings provided in the outside face of the belt, between which the rear portion of the first loop passes so that the first loop can slide vertically through said two openings over a distance D equal to H - h.

11. A lumbar support device (11) according to either one of claims 1 and 4, **characterized in that** said securing and blocking means comprise:
a) at least one vertical loop (14, 15) of inside height P (P1, P2) formed by a front portion (14a) and a rear portion (14b) between which the return strap (13) is freely arranged;
b) first fastener means (17) arranged on said front portion (14a) inside said loop (14, 15); and
c) second fastener means (18) arranged on the face of said rear portion inside said loop (14, 15); and
**in that** said first and second fastener means (17, 18) are suitable for co-operating so that in an inactive position they allow the return strap (12) to move freely in vertical translation (F1) over the entire inside height P (P1, P2) of the loop (14, 15), and in an active position, they prevent said movement in vertical translation (F1), at least in part, while allowing the return strap to move in transverse translation (F2).

12. A lumbar support device (11) according to claim 11, **characterized in that** the rear portion of the loop (14, 15) is formed by the outside face (13a) of the belt (12).

13. A lumbar support device (1, 11) according to any one of claims 5 to 12, **characterized in that** the first and second fastener means (42b, 52b, 17; 7, 18) are fastener systems of the hook-and-loop, or the press stud, or the magnet, or the repositionable adhesive type.

14. A lumbar support device (1, 11) according to any one of claims 5 to 13, **characterized in that** the belt (2, 13) includes reinforcing elements (8, 16) such as spring steel or plastics stays forming or including on their outside faces the second fastener means (7, 18).

15. A lumbar support device (1, 11) according to any one of claims 1 to 14, **characterized in that** the first and second fastener members (2c, 2d; 3c, 3d) are of the hook and loop type, in particular of the hook and unbrushed loop type.
